# EUROPEAN PATENT SPECIFICATION

(11) **EP 1 714 650 B1**
(45) Date of publication and mention of the grant of the patent: **18.08.2010**
(21) Application number: 06251848.5
(22) Date of filing: 31.03.2006
(51) Int. Cl.: A61K 31/198, A23L 1/305, A61P 25/28, A61P 21/02, A61P 43/00

(54) **Arginine-containing compositions for increasing blood flow**
Argininhaltige Zusammensetzungen zur Erhöhung des Blutflusses
Compositions contenant de l'arginine pour augmenter le flux sanguin

(30) Priority: 31.03.2005 JP 2005103590
(43) Date of publication of application: 25.10.2006
(73) Proprietor: Ajinomoto Co., Inc., Tokyo 104-8315 (JP)
(72) Inventor: Ohta, Fumio, Kawasaki-shi Kanagawa 210-8681 (JP); Sato, Hiroyuki, Kawasaki-shi Kanagawa 210-8681 (JP); Takagi, Tomo, Kawasaki-shi Kanagawa 210-8681 (JP)
(74) Representative: Nicholls, Kathryn Margaret

(56) References cited:
- WO-A-95/18608
- US-A1- 2003 018 076
- US-A1- 2003 032 616
- US-A1- 2004 092 592
- PATENT ABSTRACTS OF JAPAN vol. 2003, no. 12, 5 December 2003 (2003-12-05) & JP 2004 262878 A (MEIJI SEIKA KAISHA LTD), 24 September 2004 (2004-09-24)
- BODE-BOEGER S M ET AL: "L-arginine-induced vasodilation in healthy humans: Pharmacokinetic-pharmacodynamic relationship" BRITISH JOURNAL OF CLINICAL PHARMACOLOGY, BLACKWELL SCIENTIFIC PUBL, GB, vol. 46, no. 5, November 1998 (1998-11), pages 489-497, XP002361747 ISSN: 0306-5251
- PATENT ABSTRACTS OF JAPAN vol. 1998, no. 01, 30 January 1998 (1998-01-30) & JP 09 241156 A (NOEVIR CO LTD), 16 September 1997 (1997-09-16)
- GUPTA V ET AL: "Anti-stress and adaptogenic activity of L-arginine supplementation" EVIDENCE-BASED COMPLEMENTARY AND ALTERNATIVE MEDICINE 2005 UNITED KINGDOM, vol. 2, no. 1, March 2005 (2005-03), pages 93-97, XP002397631 Published online 8. December 2004 (8.12.2004) ISSN: 1741-427X
- CLARK R H ET AL: "NUTRITIONAL TREATMENT FOR ACQUIRED IMMUNODEFICIENCY VIRUS-ASSOCIATED WASTING USING BETA-HYDROXY BETA-METHYLBUTYRATE, GLUTAMINE, AND ARGININE: A RANDOMIZED, DOUBLE-BLIND, PLACEBO-CONTROLLED STUDY" JOURNAL OF PARENTERAL AND ENTERAL NUTRITION, AMERICAN SOCIETY FOR PARENTERAL AND ENTERAL NUTRITION, US, vol. 24, no. 3, May 2000 (2000-05), pages 133-139, XP009004307 ISSN: 0148-6071
- PATENT ABSTRACTS OF JAPAN vol. 1996, no. 12, 26 December 1996 (1996-12-26) & JP 08 198748 A (AJINOMOTO CO INC), 6 August 1996 (1996-08-06)
- PATENT ABSTRACTS OF JAPAN vol. 2000, no. 24, 11 May 2001 (2001-05-11) & JP 2001 187736 A (ITO EN LTD), 10 July 2001 (2001-07-10)
- ROTH E: "L-arginine-nitric oxide metabolism. Glutamine: A new player in this metabolic game?" CLINICAL NUTRITION, CHURCHILL LIVINGSTONE, LONDON, GB, vol. 17, no. 1, February 1998 (1998-02), pages 1-2, XP004680408 ISSN: 0261-5614
- LITCHFORD ET AL: "Use of arginine and glutamine supplements to enhance wound healing in a long-term care (LTC) resident" JOURNAL OF THE AMERICAN DIETETIC ASSOCIATION, THE ASSOCIATION, CHICAGO, IL, US, vol. 101, no. 9, September 2001 (2001-09), pages A-49, XP005107296 ISSN: 0002-8223

## Description

The present invention relates to arginine-containing compositions for increasing blood flow. Specifically, it relates to the arginine containing compositions for increasing blood flow that are useful as pharmaceutical compositions, food and beverages, feeding stuff and the like, and can increase blood flow in capillaries without lowering the systemic blood pressure.

Blood bears the transport of oxygen, nutrients, signaling molecule such as hormones, waste products, and immune cells. Namely, substances provided to blood from a lung, digestive tracts, and endocrine organs are swept away by the heartbeat and transported to the entire body through capillaries which are supplied to the body. Further, the waste products and the like are carried out into blood through the capillaries of the entire body and excreted from the organs such as a lung and a kidney. Blood also performs a certain function in heat retention in the body by circulating the blood that is always maintained at a constant temperature. Thus, blood flow of the capillaries has an important role in maintaining vital functions, but it weakens when the body bears certain loads such as diseases like infections and injuries or fatigue; aging; and sudden change of the environment. For example, as the possibility is pointed out that weakening of the blood flow of the brain relates to cerebrovascular dementia and Alzheimer-type dementia, the weakening of blood flow deteriorates the vital functions (Non-Patent Literature 1). Accordingly, it is important to actively increase blood flow when the body bears the loads, in terms of enhancing functions such as the heat retention in the body and the transport of the substances to actively enhance the vital functions.

As such agents for increasing blood flow, calcium antagonists and cellular respiratory activators, antispasm drugs, *in vivo* enzymes and the like have been used.

Since these agents for increasing blood flow also contain substances developed for lowering blood pressure and affect the blood vessels of the entire body, there is a possibility of not only increasing blood flow in the capillaries but also affecting the systemic blood pressure. In addition to it, some of the agents have other actions such as pain generation, which *in vivo* enzymes like kallikrein do. Therefore, it has been strongly desired to develop the method for increasing blood flow that is easy to use and excellent in safety.

It has been known from the prior studies that arginine produces vasodilation. As its mode of action, it has been clarified that nitric monoxide synthetases which are present in vascular endothelial cells synthesize nitric monoxide causing vasodilation from arginine. An externally administered arginine immediately synthesizes nitric monoxide and this action produces vasodilation. However, a dietary derived arginine does not usually provide such action (Non-Patent Literature 2).

Though the above action by ingestion of arginine has been widely known, is has been conventionally thought that the vasodilation by arginine causes reduction of blood pressure as well as the increase of blood flow. For example, it has been reported that, in the examination wherein 30g or 6g of arginine was parenterally administered to human beings having an average weight of 78kg, the administration of 30g of arginine lowered blood pressure and vascular resistance, while the administration of 6g of arginine did not cause either of them (Non-Patent Literature 3). In addition to this, other methods for increasing blood flow by arginine have been reported. However, in every case, dosage of arginine is overmuch or it is needed to be combined with compositions for increasing blood flow having the action for increasing blood flow by itself such as polyphenols (Non-Patent Literature 4, Patent Literature 1, 2). Therefore, it has been thought to be difficult to increase blood flow by arginine itself without lowering blood pressure.
[Patent Literature 1] United States Patent Application Publication No. 2002182162.
[Patent Literature 2] Japanese Patent Unexamined Publication No. 2004-262878.
[Non-Patent Literature 1] Biomedical Gerontology 2001: 25(2); 83-88.
[Non-Patent Literature 2] Folia Pharmacologica Japonica 2002: 119; 7-14.
[Non-Patent Literature 3] British Journal of Pharmacology 1998; 46: 489-497.
[Non-Patent Literature 4] Anesthesiology 1994; 80: 1320-1327.

The object of the present invention is to provide a composition for increasing blood flow which reduces side effects such as reduction of blood pressure and effectively increases blood flow in the capillaries.

The inventors carefully observed the changes of blood pressure and blood flow in the capillaries when administering arginine, and found that administration of arginine in a certain range of dosage increased blood flow in the capillaries of peripheral organs without lowering blood pressure. The present invention has been completed based of this finding.

Namely, the present invention provides a composition containing arginine as active ingredient, and further comprising an amino acid, or a salt thereof, the amino acid being selected from glutamine, a pyrrolidone carboxylic acid, and glutamic acid, for use in increasing blood flow in capillaries, the composition being arranged for administration of arginine in an amount of from 25mg/kg to 50mg/kg body weight per dose.

The present invention also provides a pharmaceutical composition containing the composition for use in increasing blood flow for use in preventing, diminishing or treating dementia, sensitivity to cold, stiff shoulders, dullness, or muscular fatigue.

The composition of the present invention may be in the form of a food for increasing blood flow

The composition of the present invention may, alternatively be in the form of a feeding stuff containing the composition for increasing blood flow.

According to the present invention, blood flow in the capillaries can be effectively increased while inhibiting the side effects such as reduction of the systemic blood pressure.

Arginine which can be used in the present invention include L-arginine and halide thereof and preferably chlorides thereof; L-arginine derivatives which are promptly metabolized to L-arginine *in vivo* such as the derivative forms of acid anhydrides, esters, amides, peptides, and proteins; and peptides containing L-arginine as a constituent amino acid. L-Arginine is preferable among them.

The content of arginine in the compositions for increasing blood flow of the present invention ranges from 25mg/kg body weight to 50 mg/kg body weight per dose.

The content of arginine in the compositions for increasing blood flow of the present invention is preferably the dosage which increases the concentration of free arginine in the blood plasma when administering arginine by 15mg to 180mg, preferably 25mg to 180mg, more preferably 50mg to 180mg, and yet more preferably 50mg to 150mg per 1mL of the blood plasma. The administered dose of the present invention satisfies the above content. The concentration of the free arginine in the blood plasma can be determined by taking the blood plasma 10 minutes after administering arginine, and using an amino acid analyzer (L8500, the amino acid analyzer produced by Hitachi, Ltd.)

By containing arginine in the above range, blood flow can be effectively increased in the capillaries while inhibiting side effects such as reduction of the systemic blood pressure.

In the present invention, when the blood flow increases 20% or more relative to the blood flow before the composition of the present invention is administered, it is regarded that the blood flow is increased. When the variation of blood pressure is within ± 11mmHg relative to the blood pressure before the composition of the present invention is administered, it is regarded as no variation. It is attributable to the fact that the variation of the blood flow of control sometimes indicates less than 19% and the variation of blood pressure thereof sometimes indicates less than 12mmHg.

The compositions for increasing blood flow of the present invention further contain an amino acid(s) selected from the group consisting of glutamine, a pyrrolidone carboxylic acid, glutamic acid, and salts thereof. Glutamine is particularly preferable among them.

Glutamine which can be used in the present invention includes L-glutamine, L-glutamine derivatives which are promptly metabolized to L-glutamine *in vivo* such as N-acetyl-L-glutamine, and peptide containing L-glutamine as a constituent amino acid. L-glutamine is preferable among them.

A pyrrolidone carboxylic acid which can be used in the present invention is preferably L-form. Said acid may form salts with alkali metals such as sodium and potassium, and alkaline earth metals such as calcium; basic amino acids such as arginine and lysine; or amines such as triethanolamine. Further, said acid may form derivatives with alcohols and may be in the forms of acid anhydrides, peptides, and proteins, which are constituted from said acid. Sodium salt is preferable among them.

A glutamic acid which can be used in the present invention includes L-glutamic acid and peptides containing L-glutamic acid as the constituent amino acid. Said acid may form salts with alkali metals such as sodium and potassium, and alkaline earth metals such as calcium; basic amino acids such as arginine and lysine; or amines such as triethanolamine. Further, said acid may form derivatives with alcohols and may be in the forms of acid anhydrides, peptides, and proteins constituted from said acid. L-glutamine is preferable among them.

The dosage of amino acids selected from the group consisting of glutamine, a pyrrolidone carboxylic acid, glutamic acid, and salts or derivatives thereof of the present invention is not particularly limited. However, they are preferably administered in an amount ranging from 6.25mg/kg body weight to 600mg/kg body weight per a dose, and more preferably 25 to 300mg/kg body weight per a dose. The mixed ratio of arginine to amino acids selected from the group consisting of glutamine, a pyrrolidone carboxylic acid, glutamic acid, and salts or derivatives thereof in the compositions for increasing blood flow of the present invention is preferably 1:4 to 4=1 and more preferably 1:2 to 2:1, by weight. It is preferable to contain arginine and the amino acids selected from the group consisting of glutamine, a pyrrolidone carboxylic acid, glutamic acid, and salts or derivatives thereof together in the above ratio, because volume of blood flow significantly increases.

The compositions for increasing blood flow of the present invention can be preferably used for preventing, diminishing or treating cerebrovascular dementia, Alzheimer-type dementia, stiff shoulders, sensitivity to cold, dullness, or muscular fatigue. They are excellently effective in increasing blood flow in peripheral organs, particularly in skin, muscles, bowels, heart and brain. Especially in the capillaries thereof, the compositions can effectively increase blood flow with inhibiting the reduction of the systemic blood pressure, and, therefore, distribute blood flow in the body.

In addition to the form of pharmaceutical compositions, the compositions for increasing blood flow of the present invention can be in the forms of foods, health foods, nutritional foods (supplements), nutritional compositions, or feeding stuffs, on which it is indicated that they are used for increasing blood flow. The dosage usually varies depending on the body weight, the health condition and the like of administered objects. In case of administering them to adults, it is preferably 4.5g to 27g and more preferably 9g to 27g of arginine per day.

In case of the form of the pharmaceutical compositions, they can be combined with pharmaceutically acceptable carriers or diluents, e.g., cellulose derivatives such as carboxymethylcellulose and ethylcellulose; starches such as potato starch and corn starch; sugars such as lactose and sucrose; vegetable oils such as peanut oil, corn oil and sesame oil; polyethylene glycol; alginic acid; gelatin; and tarc, to form oral agents such as tablets, dispersants, pills, granules, capsules, and syrups; injectable agents such as subcutaneously injectable agents, intravenously injectable agents, intramuscular injectable agents, peridural space injectable agents, and intrathecally injectable agents; external agents such as intranasal preparation, transdermal preparation, and ointments; suppositories such as rectal suppositories and vaginal suppositories; and intravenous fluids.

The pharmaceutical compositions of the present invention can further contain other active substances used in the pharmaceutical products such as agents for the central nerves, agents for the peripheral nerves, agents for circulatory organs, hormonal agents, antihormonal agents, vitamin preparation, revitalizers, detoxicating agents, antitumor agents, agents against allergies, galenicals, Chinese herbs, chemotherapeutic agents, biologicals, and diagnostic agents.

The pharmaceutical compositions of the present invention can orally or parenterally administered through, for example, bowels or veins.

As for food forms, it can be used as nonconventional food forms such as supplements and like, and it is also usable in the form of the usual foods. Further, they can be prepared by using a proper additive(s) in the above foods in accordance with the ordinary method(s). Examples of the additives include those usually used as the substances of health foods, such as fruit juice for adjusting and enhancing the palatability, dextrin, cyclic oligosaccharide, sugars (fructose and glucose syrup, sucrose), acidifiers, flavoring agents, green tea powder, fat and fatty oils, emulsifying agents for improving the texture, collagen, whole milk powder, polysaccharide thickeners, and agar (in case of jelly beverages).

The foods of the present invention can be further prepared as the health foods by dispensing amino acids, vitamins, eggshell calcium, calcium pantothenate, other minerals, royal jelly, propolis, honey, dietary fibers, agaricus, chitin, chitosan, capsaicin, polyphenol, carotenoid, fatty acids, mucopolysaccharides, coenzymes, and antioxidants.

The compositions for increasing blood flow of the present invention may also be prepared as feeding stuffs for mammals such as swines, bovines, sheep, canines, felines, mice, rats, and simians. For example, it can be prepared as solid or liquid additives for feeding stuffs in accordance with the known methods of the aforementioned technical field.

The product forms of the compositions for increasing blood flow of the present invention are not particularly limited, and any forms are possible only if they are those from which usually used amino acids are ingestible. In case of oral administration, examples of such forms include powder, granules, tablets, liquid (beverages, jelly beverages), and candies (such as chocolates), wherein a suitable excipient(s) is used; or the mixture of one or two kinds of the above amino acids. In case of intravenous administration, they include transfusions and aqueous solutions containing one or two kinds of the above amino acids, and amino acid powder added before using.

On the measurement of blood flow in the capillaries, which is an important outcome measure in the present invention, the measurement can be conducted by using a laser Doppler blood-flowmeter (FLO-N1 produced by OMEGAWAVE, INC.) or a microsphere (DYE-TRAK VII+ produced by Triton Technology Ltd.) or the like. Further, changes in the delivery amounts of the compositions to various organs due to the increase of blood flow can be determined by the distribution quantities of intravenously administered pigments such as Evans' Blue to the various organs. Meanwhile, though Evans' Blue itself is not a medicament, it has been reported that the delivery amounts of the compositions such as medicaments can be estimated by the above method (Non-Patent Literature 5). Namely, Evans' Blue is a pigment having a molecular weight of 960.08, and its evaluation as a pharmaceutical model molecule is established on examining the delivery of the medicaments.

On the other hand, as for the measurement of blood pressure, blood pressure in the catheter inserted to the arteries can be measured over time by using a pressure sensor (Transducer TP-400T produced by Nihon Kohden Corporation), an amplifier (AP-601G produced by Nihon Kohden Corporation), and a recorder (MacLab/16S produced by ADInstruments).
[Non-Patent Literature 5] Journal of Neurosurgery 2004; 101: 303-309

### Examples

Next, Test Examples will further illustrate embodiments of the present invention.

### Test Example 1 (Checking variations of blood pressure and blood flow when administering arginine to rats) (Reference Example).

### (1) Outline of the test:

(a) The changes of the systemic blood pressure and the effect of increasing the blood flow in the capillaries of the skin of lower thighs were examined on rats when administering arginine.
(b) The subjects were male SD rats having a body weight of around 400g.
(c) Rats were anesthetized by pentobarbital and a catheter was inserted to their arteries. Then, the blood pressure thereof was measured and recorded over time. Further, a probe(s) of a laser Doppler blood-flowmeter was fixed on the skin of the lower thighs, and the blood flow of the skin surface was measured and recorded over time. Arginine was adjusted to pH 7.4 with a citric acid and continuously injected at a constant speed through the jugular vein in the dosages of 50, 150 and 500mg per 1kg of the body weight over 30 minutes. The blood pressure before and after the treatment and the blood flow of the skin of the lower thighs were observed. Three rats were used per one administration dose and the evaluation was conducted by the variation of the average values of the three rats. A saline solution was administered to a negative objective group.
(d) After the administration of arginine, the amount of blood of the skin of lower thighs increased in all rats to which 50mg or more of arginine per 1kg of the body weight was administered. As a result, it was confirmed that the blood flow increased. The reduction of blood pressure beyond 11mmHg was not observed in rats to which 150mg or less of arginine per 1kg of the body weight was administered. However, the reduction of blood pressure was observed in rats to which 500mg or more of arginine per 1kg of the body weight was administered. Two out of three rats died due to the rapid reduction of the blood pressure.
(e) From the above results, while the reduction of blood pressure and the increase of blood flow were observed in the rats to which 500mg of arginine per 1kg of the body weight was administered, the increase of blood flow with hardly lowering blood pressure was observed in the rats to which 150mg or less of arginine per 1kg of the body weight was administered. Thus, it was thought that the administration of arginine in the amount defined in the present invention caused the increase of blood flow with hardly lowering blood pressure.

### (2) Details of the test

(a) Constitution of each administration groups: See the following Table 1.

**Table 1**

| Administration groups | Composition | Arginine concentration and admin'd amount of the admin'd solution |
|---|---|---|
| Control group | A saline solution | 0mg/mL |
| | | 5mL/kg/30mins. |
| 50mg of arginine per 1kg of the body weight admin'd group | Arginine, Citric acid | 10mg/mL |
| | | 5mL/kg/30mins. |
| 150mg of arginine per 1kg of the body weight admin'd group | Arginine, Citric acid | 30mg/mL |
| | | 5mL/kg/30mins. |
| 500mg of arginine per 1kg of the body weight admin'd group | Arginine, Citric acid | 100mg/mL |
| | | 5mL/kg/30mins. |

(b) Measurement of blood pressure: The blood pressure in the catheter inserted to the arteries was measured and recorded over time by using a pressure sensor (Transducer TP-400T produced by Nihon Kohden Corporation), an amplifier (AP-601G produced by Nihon Kohden Corporation), and a recorder (MacLab/1GS produced by ADInstruments).
(c) Measurement of blood flow: A thin probe(s) connected to a laser Doppler blood-flowmeter (FLO-N1 produced by OMEGAWAVE, INC.) was attached to the skin surface. Then, the amount of blood, the speed thereof and the blood flow were measured by irradiating a laser.
(d) Test Results: The following Table 2 shows changes in the blood pressure and the blood flow which were observed in the arginine administration test. The blood pressure and the blood flow were measured right before starting to inject arginine, 20 to 30 minutes after starting the injection (in case of injecting the dosages of 50mg or 150mg), and 50 to 60 minutes after starting the injection (in case of injecting the dosage of 500mg), and indicated as average values of the values during 10 minutes.

**Table 2**

| Administration groups | Maximum variation of blood pressure | Maximum increasing rate of blood flow (Before administration: 100%) |
|---|---|---|
| Control group | -11mmHg | +9.47% |
| 50mg of arginine per 1kg of the body weight admin'd group | -6mmHg | +43.9% |
| 150mg of arginine per 1kg of the body weight admin'd group | -8mmHg | +29.3% |
| 500mg of arginine per 1kg of the body weight admin'd group | -118mmHg | +85.0% |

### Test Example 2 (Measurement of blood flow in the capillaries of various organs with the microsphere method) (Reference Example)

### (1) Outline of the test:

(a) The changes of blood flow in the capillaries of several organs were examined on rats when administering arginine.
(b) The subjects were male SD rats having a body weight of around 400g.
(c) Rats were anesthetized by pentobarbital. Then, arginine was adjusted to pH 7.4 with a citric acid and continuously injected at a constant speed through the jugular vein in the dosages of 0, 50 or 150mg per 1kg of the body weight over 30 minutes. Before and after such administration of arginine, each of yellow and red microspheres were injected from the catheter placed in the left ventricle through the jugular vein. Then, the blood flows of each organ before and after the administration of arginine were compared by comparing the amounts of the microspheres distributed from the heart to the capillaries of the organs of the entire body.
(d) In all organs measured this time, that is, muscles, digestive tracts, liver, kidneys, spleen, heart, brain, adipose tissues, and abdominal skin, the tendency was observed that blood flow in the capillaries increased depending on the dosage of arginine. In the rats to which 150mg of arginine per 1kg of the body weight was administered, the blood flow in the capillaries of all organs measured this time increased. On the other hand, even in the rats to which 50mg of arginine per 1kg of the body weight was administered, it was confirmed that the blood flow in the capillaries of the muscles and the brain significantly increased.
(e) From the above results, the increase of blood flow in the capillaries of wide range of organs was shown in the rats to which 150mg of arginine per 1kg of the body weight was administered. On the other hand, the increase of blood flow in the capillaries of the muscles and the brain was clearly shown in the rats to which 50mg of arginine per 1kg of the body weight was administered. Thus, it was thought that the administration of arginine in the dosage of the present invention was particularly notable in the muscles and the brain.
(f) As for blood pressure, the variation thereof was within 10mmHg. Therefore, it was shown that blood pressure did not change when administering 50 or 150mg of arginine per 1kg of the body weight.

### (2) Details of the test

(a) Constitution of each administration groups: See the following Table 3.

**Table 3**

| Administration groups | Composition | Arginine concentration and admin'd amount of the admin'd solution |
|---|---|---|
| Control group | A saline solution | 0mg/mL 5mL/kg/30mins. |
| 50mg of arginine per 1kg of the body weight admin'd group | Arginine, Citric acid | 10mg/mL 5mL/kg/30mins. |
| 150mg of arginine per 1kg of the body weight admin'd group | Arginine, Citric acid | 30mg/mL 5mL/kg/30mins. |

(b) Operation: Catheters were inserted to the carotid artery, jugular vein, and femoral artery of rats under anesthesia. Then, the microsphere and arginine were administered respectively, and the sample blood was collected from each catheters.
(c) Measurement of blood flow: Right before and just after 30-minute administration of arginine, yellow and red microspheres were injected from the catheter placed in the left ventricle. After taking enough time from the second administration of the microsphere, the rats were put euthanasia, and the muscles, digestive tracts, liver, kidneys, spleen, heart, brain, adipose tissues, and abdominal skin were taken out from them. Then, the microspheres contained in each organ were collected and the rate of change in blood flow was calculated with each organ from the amount of the microsphere.
(d) Test Results: The following Table 4 shows changes in blood flow which were observed in the arginine administration test.

**Table 4**

| Changes in blood flow after the administration of arginine (Variation before the administration is 100%) | | | |
|---|---|---|---|
| | 0mg/kg of body weight | 50mg/kg of body weight | 150mg/kg of body weight |
| Muscles | 102% | 164% | 157% |
| Abdominal skin | 95% | 117% | 134% |
| Back skin | 95% | 107% | 118% |
| Adipose tissues | 87% | 99% | 116% |
| Kidneys | 68% | 86% | 91% |
| Spleen | 96% | 104% | 115% |
| Duodenum | 109% | 123% | 142% |
| Liver | 65% | 92% | 101% |
| Heart | 121% | 126% | 140% |
| Brain | 115% | 126% | 137% |

### Test Example 3 (Measurement of the concentration of free arginine in the blood plasma when intravenously administering arginine) (Reference Example)

### (1) Outline of the test:

(a) The concentration of free arginine in the blood plasma was examined on rats when intravenously administering arginine.
(b) The subjects were male SD rats having a body weight of around 400g.
(c) Rats were anesthetized by pentobarbital. Then, arginine was adjusted to pH 7.4 with a citric acid and continuously injected at a constant speed through the jugular vein in the dosages of 0, 50 or 150mg per 1kg of the body weight over 30 minutes starting from 10:00a.m. Right before such administration of arginine and every 10 minutes from 10 to 40 minutes after the end of the administration, blood was collected from the catheter placed in the femoral artery. Then, the concentration of free arginine in the blood plasma was measured.
(d) In the rats to which 50mg of arginine per 1kg of the body weight was administered, the concentration of free arginine in the blood plasma rose by 46.1 micrograms per 1mL at maximum. In the rats to which 150mg of arginine per 1kg of the body weight was administered, the concentration of free arginine in the blood plasma rose by 177.5 micrograms per 1mL at maximum. On the other hand, in the control group to which arginine was not administered, the concentration of free arginine in the blood plasma did not rise.
(e) From the above results, when administering 50 or 150mg of arginine per 1kg of the body weight, by which the increase of blood flow was observed, it was confirmed that each of the concentrations of free arginine in the blood plasma rose by 15.7 to 46.1 micrograms per 1mL or 77.2 to 177.5 micrograms per 1mL.
(f) As for blood pressure, Test Example 1, wherein the same amounts of arginine were administered, shows that the blood pressure did not change. Therefore, it was confirmed that blood pressure was not changed by the variation of the concentrations of arginine in the blood plasma within the above range.

### (2) Details of the test

(a) Compositions of each administered solutions: See the following Table 5.

**Table 5**

| Administration groups | Composition | Arginine concentration and admin'd amount of the admin'd solution |
|---|---|---|
| Control group | A saline solution | 0mg/mL |
| | | 5mL/kg/30mins. |
| 50mg of arginine per 1kg of the body weight admin'd group | Arginine, Citric acid | 10mg/mL |
| | | 5mL/kg/30mins. |
| 150mg of arginine per 1kg of the body weight admin'd group | Arginine, Citric acid | 30mg/mL |
| | | 5mL/kg/30mins. |

(b) Administration: An aqueous solution wherein arginine was dissolved was continuously injected at a constant speed through the catheter placed in the jugular vein in the dosages of 50 or 150mg per 1kg of the body weight over 30 minutes.
(c) Measurement of the concentration of free arginine in the blood plasma: Before and after the administration of arginine, the rats' blood was collected from the catheter placed in their femoral arteries. Then, the concentration of free arginine contained in the blood was measured with an amino acid analyzer (L8500, the amino acid analyzer produced by Hitachi, Ltd.)
(d) Test results: The following Table 6 shows changes over time in the amounts of rise of the concentration of free arginine in the blood plasma which were observed in the arginine administration test.

**Table 6**

| Average values of the amounts of rise of the concentration of free arginine in the blood plasma (unit: µg/mL) | | | | |
|---|---|---|---|---|
| Administration groups | 10 minutes later | 20 minutes later | 30 minutes later | 40 minutes later |
| Control group | 0.2 | 0.5 | 0.4 | 0.6 |
| 50mg of arginine per 1kg of the body weight admin'd group | 43.6 | 46.1 | 38.8 | 15.7 |
| 150mg of arginine per 1kg of the body weight admin'd group | 155.5 | 114.8 | 177.5 | 77.2 |

As seen in the administration of 50 or 150mg of arginine per 1kg of the body weight, by which the increase of blood flow was observed, the range of rise of the concentration of free arginine in the blood plasma was about 15 to 180 micrograms per 1mL of blood plasma. Thus, it was shown that the rise of the concentration of free arginine in the blood plasma which was necessary for increasing blood flow was within the above range.

### Test Example 4 (Checking variations of blood pressure and blood flow when administering arginine and glutamine to rats)

### (1) Outline of the test:

(a) The changes of the systemic blood pressure and the effect of increasing the blood flow in the capillaries of the skin of lower thighs were examined on rats when administering arginine and glutamine.
(b) The subjects were male SD rats having a body weight of around 400g.
(c) Rats were anesthetized by pentobarbital and a catheter was inserted to their arteries. Then, the blood pressure thereof was measured and recorded over time. Further, a probe(s) of a laser Doppler blood-flowmeter was fixed on the skin of the lower thighs, and the blood flow of the skin surface was measured and recorded over time. As for an administered solution, arginine neutralized with a citric acid or the solution mixed with equal quantities of arginine and glutamine was used and continuously injected at a constant speed in the dosage of 50mg of arginine per 1kg of the body weight over 30 minutes. The blood pressure and the blood flow of the skin of the lower thighs before and after the treatment were observed. Three rats were used per one administration dose and the evaluation was conducted by the variation of the average value of the three rats. A saline solution was administered to a negative objective group.
(d) It was confirmed that, after the administration of arginine, the amount of the blood flow of the skin of lower thighs increased by +20.0% at maximum in all rats to which 50mg of arginine per 1kg of the body weight was administered. Meanwhile, it was confirmed that the amount of the blood flow of the skin of lower thighs increased by +39.9% at maximum in the rats to which the solution mixed with 50mg of arginine per 1kg of the body weight and equal quantity of glutamine was administered.
(e) From the above results, since the administration of the solution mixed with 50mg of arginine per 1kg of the body weight and equal quantity of glutamine particularly increased blood flow as compared with that of 50mg of arginine only per 1kg of the body weight, it was thought that the concurrent use of glutamine enhanced the increase of blood flow by the administration of arginine in the present invention.

### (2) Details of the test

(a) Constitution of each administration groups: See the following Table 7.

**Table 7**

| Administration groups | Composition | Arginine concentration and admin'd amount of the admin'd solution |
|---|---|---|
| Control group | A saline solution | 0mg/mL |
| | | 5mL/kg/30mins. |
| 50mg of arginine per 1kg of the body weight admin'd group | Arginine, Citric acid | 10mg/mL |
| | | 5mL/kg/30mins. |
| 50mg of arginine per 1kg of the body weight + 50mg of glutamine per 1kg of the body weight admin'd group | Arginine, Glutamine Citric acid | 10mg/mL |
| | | 5mL/kg/30mins. |

(b) Measurement of blood pressure: The blood pressure in the catheter inserted to the arteries was measured and recorded over time by using a pressure sensor (Transducer TP-400T produced by Nihon Kohden Corporation), an amplifier (AP-601G produced by Nihon Kohden Corporation), and a recorder (MacLab/16S produced by ADInstruments).
(c) Measurement of blood flow: A thin probe(s) connected to a laser Doppler blood-flowmeter (FLO-N1 produced by OMEGAWAVE, INC.) was attached to the skin surface. Then, the amount of blood, the speed thereof and the blood flow were measured by irradiating a laser.
(d) Test Results: The following Table 8 shows changes over time in blood pressure and blood flow which were observed in the arginine administration test. The blood pressure and the blood flow were measured right before starting to inject arginine and 20 to 30 minutes after starting the injection, and indicated as average values of the values during 10 minutes.

**Table 8**

| Administration groups | Maximum variation of blood pressure | Maximum increasing rate of blood flow (Before administration: 100%) |
|---|---|---|
| Control group | -5.38mmHg | +9.47% |
| 50mg of arginine per 1kg of the body weight admin'd group | +11.4mmHg | +20.0% |
| 50mg of arginine per 1kg of the body weight + 50mg of glutamine per 1kg of the body weight admin'd group | +22.1mmHg | +39.9% |

### Test Example 5 (Measurement of the delivery amounts of the compositions to various organs by using Evans' Blue) (Reference Example)

### (1) Outline of the test:

(a) The changes of the delivery amounts of Evans' Blue (Wako Pure Chemical Industries, Ltd.) which was used as a model for estimating the delivery amounts of compositions such as pharmaceutical compositions were measured in order to examine the changes of the delivery amounts of the compositions to several organs of rats when administering arginine.
(b) The subjects were male SD rats having a body weight of around 400g.
(c) Rats were anesthetized by pentobarbital. Then, arginine was adjusted to pH 7.4 with a citric acid and continuously injected at a constant speed through the jugular vein in the dosages of 0, 50 or 150mg per 1kg of the body weight over 30 minutes. Before and after such administration of arginine, Evans' Blue was injected from the catheter placed in the jugular vein. Then, the delivery amounts of the compositions to each organs were compared by comparing the amounts of Evans' Blue distributed to the organs of the entire body with a spectrophotometer.
(d) In muscles, digestive tracts, liver, kidneys, spleen, brain, adipose tissues, and skin measured this time, the tendency was observed that the delivery amounts of Evans' Blue to the organs increased in the dosage of 50 or 150mg of arginine per 1kg of the body weight.
(e) From the above results, in the rats to which 50mg of arginine per 1kg of the body weight was administered, the delivery amount of Evans' Blue increased. Thus, it was shown that the delivery amounts of the compositions to organs were increased by the administration of arginine in the dosage of the present invention.

### (2) Details of the test

(a) Constitution of each administration groups: See the following Table 9.

**Table 9**

| Administration groups | Composition | Arginine concentration and admin'd amount of the admin'd solution |
|---|---|---|
| Control group | A saline solution | 0mg/mL |
| | | 5mL/kg/30mins. |
| 50mg of arginine per 1kg of the body weight admin'd group | Arginine, Citric acid | 10mg/mL |
| | | 5mL/kg/30mins. |
| 150mg of arginine per 1kg of the body weight admin'd group | Arginine, Citric acid | 30mg/mL |
| | | 5mL/kg/30mins. |

(b) Operation: A catheter was inserted to the jugular vein of rats under anesthesia. Then, the sample blood was collected from the catheter.
(c) Measurement of the delivery amounts of Evans' Blue: An aqueous solution of Evans' Blue (50mg/mL) was injected through the catheter placed in the jugular vein just after administering arginine over 30 minutes. After taking enough time, the rats were put euthanasia, and the muscles, digestive tracts, liver, kidneys, spleen, heart, brain, adipose tissues, and abdominal skin were taken out from them. Then, Evans' Blue was dissolved out by immersing each organ in a formalin solution, and the delivery amounts of Evans' Blue of each organs were calculated from the absorbance obtained by the spectrophotometer.
(d) Test results: The following Table 10 shows changes in the delivery amounts of Evans' Blue which were observed in the arginine administration test.

**Table 10**

| Changes in the delivery amounts of Evans' Blue after administering arginine (Variation before the administration is 100%) | | |
|---|---|---|
| | 50mg/kg of BW | 150mg/kg of BW |
| Gastrocnemius muscle | 219% | 154% |
| Soleus muscle | 123% | 125% |
| Abdominal skin | 124% | 207% |
| Back skin | 315% | 331% |
| Adipose tissues | 430% | 160% |
| Kidneys | 161% | 128% |
| Spleen | 101% | 92% |
| Duodenum | 143% | 85% |
| Liver | 181% | 173% |
| Heart | 87% | 85% |
| Brain | 169% | 128% |

## Claims

1. A Composition containing arginine as active ingredient, and further comprising an amino acid, or a salt thereof, the amino acid being selected from glutamine, a pyrrolidone carboxylic acid, and glutamic acid, for use in increasing blood flow in capillaries, the composition being arranged for administration of arginine in an amount of from 25mg/kg to 50mg/kg body weight per dose.

2. A composition for use according to claim 1, wherein the arginine is selected from the group consisting of L-arginine, L-arginine chlorides and L-arginine derivatives which are metabolized to L-arginine *in vivo*, which L-arginine derivatives are selected from acid anhydrides, esters, amides, proteins and peptides containing L-arginine as a constituent amino acid.

3. A composition for use according to claim 1 or 2, wherein the amino acid is selected from:
L-glutamirie; and derivatives metabolized to L-glutamine *in vivo* selected from N-acetyl-L-glutamine, and peptides containing L-glutamine as a constituent amino acid;
L-pyrrolidone carboxylic acid; alkali metal and alkaline earth metal salts of pyrrolidone carboxylic acid; salts of pyrrolidone carboxylic acid with basic amino acids; amine salts of pyrrolidone carboxylic acid; derivatives of pyrrolidone carboxylic acid with alcohols; acid anhydrides, peptides and proteins including pyrrolidone carboxylic acid; and
L-glutamic acid; peptides containing L-glutamzc acid; alkali metal and alkaline earth metal salts of glutamic acid; salts of glutamic acid with basic amino acids and amine salts of glutamic acid; acid anhydrides, peptides and proteins including glutamic acid.

4. The composition for use according to any one of claims 1 to 3, wherein the ratio of arginine to the amino acids selected from the group consisting of glutamine, a pyrrolidone carboxylic acid, glutamic acid, and salts or derivatives thereof is 1:4 to 4:1 by weight.

5. The composition for use according to any one of the preceding claims, wherein the amino acid is glutamine.

6. The composition for use according to claim 5, wherein the composition is arranged for administration of glutamine in an amount ranging from 6.25mg/kg body weight to 600mg/kg body weight per dose.

7. The composition for use according to claim 6, wherein the amount of glutamine ranges from 25mg/kg body weight to 300mg/kg body weight per a dose.

8. A composition for use according to any one of the preceding claims wherein the composition is for use in preventing, diminishing or treating dementia, sensitivity to cold, stiff shoulders, dullness, or muscular fatigue.

9. A composition for use according to any one of claims 1 to 8, wherein the composition is a food.

10. A composition for use according to any one of claims 1 to 8, wherein the composition is a feeding stuff.

11. Use of arginine as active ingredient in the manufacture of a composition for increasing blood flow in capillaries, the composition being arranged for administration of arginine in an amount of from 25mg/kg to 50mg/kg body weight per dose, the composition further comprising an amino acid, or a salt thereof, the amino acid being selected from glutamine, a pyrrolidone carboxylic acid, and glutamic acid.

12. Use according to claim 11 wherein the ratio of arginine to the amino acids selected from the group consisting of glutamine, a pyrrolidone carboxylic acid, glutamic acid, and salts or derivatives thereof is 1:4 to 4:1 by weight.

13. Use according to claim 11 or 12 wherein the amino acid is glutamine.

14. Use according to claim 13 wherein the composition is arranged for administration of glutamine in an amount ranging from 6.25mg/kg body weight to 600mg/kg body weight per dose.

15. Use according to any one of claims 11-14 wherein the composition is for use in preventing, diminishing or treating dementia, sensitivity to cold, stiff shoulders, dullness, or muscular fatigue.

## Patentansprüche

1. Zusammensetzung, die Arginin als aktiven Bestandteil enthält, und die außerdem eine Aminosäure oder ein Salz davon enthält, wobei die Aminosäure unter Glutamin, einer Pyrrolidoncarbonsäure und Glutaminsäure ausgewählt ist, für die Verwendung zum Erhöhen des Blutflusses in Kapillaren, wobei die Zusammensetzung für die Verabreichung von Arginin in einer Menge von 25 mg/kg bis 50 mg/kg Körpergewicht pro Dosis zusammengestellt ist.

2. Zusammensetzung für die Verwendung nach Anspruch 1, wobei Arginin aus der Gruppe ausgewählt ist, die aus L-Arginin, L-Argininchloriden und L-Argininderivaten besteht, die in vivo zu L-Arginin metabolisiert werden, wobei die L-Argininderivate unter Säureanhydriden, Estern, Amiden, Proteinen und Peptiden, die L-Arginin als eine Konstitutionsaminosäure enthalten, ausgewählt sind.

3. Zusammensetzung für die Verwendung nach Anspruch 1 oder 2, wobei die Aminosäure ausgewählt ist unter;
L-Glutamin und Derivaten, die in vivo zu L-Glutamin metabolisiert werden, ausgewählt unter N-Acetyl-L-glutamin, und Peptiden, die L-Glutamin als eine Konstitutionsaminosäure enthalten;
L-Pyrrolidoncarbonsäure; Akalimetall- und Erdalkalimetallsalze von Pyrrolidoncarbonsäure; Salze von Pyrrolidoncarbonsäure mit basischen Aminosäuren; Aminsalze von Pyrrolidoncarbonsäure; Derivate von Pyrrolidoncarbonsäure mit Alkoholen; Säureanhydride, Peptide und Proteine, einschließlich Pyrrolidoncarbonsäure; und
L-Glutaminsäure; Peptide, die L-Glutaminsäure enthalten; Alkalimetall- und Erdalkalimetallsalzen von Glutaminsäure; Salzen von Glutaminsäure mit basischen Aminosäuren und Aminsalzen von Glutaminsäure; Säureanhydriden, Peptiden und Proteinen, einschließlich Glutaminsäure.

4. Zusammensetzung für die Verwendung nach einem der Ansprüche 1 bis 3, wobei das Verhältnis von Arginin zu den Aminosäuren, die aus der aus Glutamin, einer Pyrrolidoncarbonsäure, Glutaminsäure und Salzen oder Derivaten davon bestehenden Gruppe ausgewählt sind, 1:4 bis 4:1 (Gewichtsverhältnis) ist.

5. Zusammensetzung für die Verwendung nach einem der vorstehenden Ansprüche, wobei die Aminosäure Glutamin ist.

6. Zusammensetzung für die Verwendung nach Anspruch 5, wobei die Zusammensetzung für die Verabreichung von Glutamin in einer Menge im Bereich von 6,25 mg/kg Körpergewicht bis 600 mg/kg Körpergewicht pro Dosis zusammengestellt ist.

7. Zusammensetzung für die Verwendung nach Anspruch 6, wobei die Menge an Glutamin im Bereich von 25 mg/kg Körpergewicht bis 300 mg/kg Körpergewicht pro Dosis liegt.

8. Zusammensetzung für die Verwendung nach einem der vorstehenden Ansprüche, wobei die Zusammensetzung zur Prävention, Linderung oder Behandlung von Demenz, Empfindlichkeit gegenüber Kälte, steifen Schultern, Mattheit und Muskelermüdung verwendet wird.

9. Zusammensetzung für die Verwendung nach einem der Ansprüche 1 bis 8, wobei die Zusammensetzung ein Nahrungsmittel ist.

10. Zusammensetzung für die Verwendung nach einem der Ansprüche 1 bis 8, wobei die Zusammensetzung ein Futtermittel ist.

11. Verwendung von Arginin als aktiven Bestandteil bei der Herstellung einer Zusammensetzung zum Erhöhen des Blutflusses in Kapillaren, wobei die Zusammensetzung für die Verabreichung von Arginin in einer Menge von 25 mg/kg bis 50 mg/kg Körpergewicht pro Dosis zusammengestellt ist, wobei die Zusammensetzung außerdem eine Aminosäure oder ein Salz davon enthält, wobei die Aminosäure unter Glutamin, Pyrrolidoncarbonsäure und Glutaminsäure ausgewählt ist.

12. Verwendung nach Anspruch 11, wobei das Verhältnis von Arginin zu den Aminosäuren, die aus der aus Glutamin, einer Pyrrolidoncarbonsäure, Glutaminsäure und Salzen oder Derivaten davon bestehenden Gruppe ausgewählt sind, 1:4 bis 4:1 (Gewichtsverhältnis) ist.

13. Verwendung nach Anspruch 11 oder 12, wobei die Aminosäure Glutamin ist.

14. Verwendung nach Anspruch 13, wobei die Zusammensetzung für die Verabreichung von Glutamin in einer Menge im Bereich von 6,25 mg/kg Körpergewicht bis 600 mg/kg Körpergewicht pro Dosis zusammengestellt ist.

15. Verwendung nach einem der Ansprüche 11 bis 14, wobei die Zusammensetzung für die Verhinderung, Linderung oder Behandlung von Demenz, Empfindlichkeit gegenüber Kälte, steifen Schultern, Mattheit und Muskelermüdung verwendet wird.

## Revendications

1. Composition contenant de l'arginine comme ingrédient actif, et comprenant en outre un aminoacide, ou un sel de celui-ci, i'aminoacide étant choisi parmi la glutamine, un acide pyrrolidone carboxylique, et l'acide glutamique, destinée à être utilisée dans l'augmentation du débit sanguin dans les capillaires, la composition étant agencée pour l'administration d'arginine en une quantité de 25 mg/kg à 50 mg/kg de poids corporel par dose.

2. Composition destinée à être utilisée selon la revendication 1, où l'arginine est choisie dans le groupe consistant en la L-arginine, les chlorures de L-arginine et les dérivés de L-arginine qui sont métabolisés en L-arginine *in vivo*, lesquels dérivés de L-arginine sont choisis parmi les anhydrides, les esters, les amides d'acide, les protéines et les peptides contenant de la L-arginine comme aminoacide constitutif.

3. Composition destinée à être utilisée selon la revendication 1 ou 2, où l'aminoacide est choisi parmi :
la L-glutamine ; et les dérivés métabolisés en L-glutamine *in vivo* choisis parmi la N-acétyl-L-glutamine, et les peptides contenant de la L-glutamine comme aminoacide constitutif ;
l'acide L-pyrrolidone carboxylique ; les sels de métaux alcalins et de métaux alcalino-terreux d'acide pyrrolidone carboxylique ; les sels d'acide pyrrolidone carboxylique avec des aminoacides basiques ; les sels d'amine et d'acide pyrrolidone carboxylique ; les dérivés d'acide pyrrolidone carboxylique avec des alcools ; les anhydrides d'acide, les peptides et les protéines incluant de l'acide pyrrolidone carboxylique ; et
l'acide L-glutamlque ; les peptides contenant de l'acide L-glutamique ; les sels de métaux alcalins et de métaux alcallno-terreux de l'acide glutamique ; les sels de l'acide glutamique avec des aminoacides basiques et les sels d'amine de l'acide glutamique ; les anhydrides d'acide, les peptides et les protéines incluant de l'acide glutamique.

4. Composition destinée à être utilisée selon l'une quelconque des revendications 1 à 3, où le rapport de l'arginine aux aminoacides choisis dans le groupe consistant en la glutamine, un acide pyrrolidone carboxylique, l'acide glutamique, leurs sels ou dérivés est 1 : 4 à 4 : 1 en poids.

5. Composition destinée à être utilisée selon l'une quelconque des revendications précédentes, où l'aminoacide est la glutamine.

6. Composition destinée à être utilisée selon la revendication 5, où la composition est agencée pour l'administration de glutamine en une quantité allant de 6,25 mg/kg de poids corporel à 600 mg/kg de poids corporel par dose.

7. Composition destinée à être utilisée selon la revendication 6, où la quantité de glutamine va de 25 mg/kg de poids corporel à 300 mg/kg de poids corporel par dose.

8. Composition destinée à être utilisée selon l'une quelconque des revendications précédentes où la composition est destinée à être utilisée dans la prévention, la diminution ou le traitement de la démence, de la sensibilité au froid, des épaules raides, de la lourdeur ou de la fatigue musculaire.

9. Composition destinée à être utilisée selon l'une quelconque des revendications 1 à 8, où la composition est un aliment.

10. Composition destinée à être utilisée selon l'une quelconque des revendications 1 à 8, où la composition est une matière d'alimentation.

11. Utilisation de l'arginine comme ingrédient actif dans la fabrication d'une composition pour augmenter le débit sanguin dans les capillaires, la composition étant agencée pour l'administration d'arginine en une quantité de 25 mg/kg à 50 mg/kg de poids corporel par dose, la composition comprenant en outre un aminoacide, ou un sel de celui-ci, l'aminoacide étant choisi parmi la glutamine, un acide pyrrolidone carboxylique et l'acide glutamique.

12. Utilisation selon la revendication 11 où le rapport de l'arginine aux aminoacides choisis dans le groupe consistant en la glutamine, un acide pyrrolidone carboxylique, l'acide glutamique et leurs sels ou dérivés est 1 : 4 à 4 : 1 en poids.

13. Utilisation selon la revendication 11 ou 12 où l'aminoacide est la glutamine.

14. Utilisation selon la revendication 13 où la composition est agencée pour l'administration de glutamine en une quantité allant de 6,25 mg/kg de poids corporel à 600 mg/kg de poids corporel par dose.

15. Utilisation selon l'une quelconque des revendications 11-14 où la composition est destinée à être utilisée dans la prévention, la diminution ou le traitement de la démence, de la sensibilité au froid, des épaules raides, de la lourdeur ou de la fatigue musculaire.
